(19)

Europäisches
Patentamt
European
Patent Office
Office européen
des brevets

(11) **EP 3 863 019 A1**

(12) **EUROPEAN PATENT APPLICATION**

(43) Date of publication:
**11.08.2021 Bulletin 2021/32**

(51) Int Cl.:
**G16B 20/20** *(2019.01)*  **G16B 40/30** *(2019.01)*

(21) Application number: **20156032.3**

(22) Date of filing: **07.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sophia Genetics S.A.**
**1025 Saint Sulpice (CH)**

(72) Inventors:
- **SONG, Lin**
  **1025 Saint-Sulpice (CH)**
- **XING, Xiaboin**
  **1025 Saint-Sulpice (CH)**
- **XU, Zhenyu**
  **1291 Commugny (CH)**

(74) Representative: **Wenger, Joel-Théophile**
**IP Partners J. Wenger**
**Rte des Avouillons 6**
**1196 Gland (CH)**

(54) **METHODS FOR DETECTING AND CHARACTERIZING MICROSATELLITE INSTABILITY WITH HIGH THROUGHPUT SEQUENCING**

(57)  The characterization, classification and reporting of MSI status of patient genomic samples may be provided by high throughput genomic analysis of a set of microsatellite marker loci having a mono-homopolymer repeat length between 13 and 25 nucleotides. The patient sample may only comprise a low fraction of somatic DNA relative to germline DNA. A multi-parametric background model may be used to infer at least two parameters respectively characterizing the sample variant fraction and the MSI genomic alterations of the patient DNA sample relative to a reference background model of the MSS homopolymer length distribution, without the need to use a germline control sample. A local MSI score may be calculated as a function of the at least two parameters to characterize the MSI status at each locus, and a global composite MSI score may be calculated over all tested loci to characterize and report the overall MSI status for the patient sample. The proposed methods facilitate the deployment of high-throughput genomic data analysis testing for large pools of patients with comparable sensitivity and specificity to prior art biological MSI-status characterization assays.

FIG.1

EP 3 863 019 A1

**Description**

FIELD OF THE INVENTION

Methods described herein relate to genomic analysis in general, and more specifically to next generation sequencing applications.

BACKGROUND OF THE INVENTION

Next-generation sequencing

*High throughput sequencing (HTS), next-generation sequencing* (NGS) or *massively parallel sequencing* (MPS) technologies have significantly decreased the cost of DNA sequencing in the past decade. NGS has broad application in biology and dramatically changed the way of research or diagnosis methodologies. For example, RNA expression profiling or DNA sequencing can only be conducted with a few numbers of genes with traditional methods, such as quantitative PCR or Sanger sequencing. Even with microarrays, profiling the gene expression or identifying the mutation at the whole genome level can only be implemented for organisms whose genome size is relatively small. With NGS technology, RNA profiling or whole genome sequencing has become a routine practice now in biological research. On the other hand, due to the high throughput of NGS, multiplexed methods have been developed not just to sequence more regions but also to sequence more samples. Compared to the traditional Sanger sequencing technology, NGS enables the detection of mutation for much more samples in different genes in parallel. Due to its superiorities over traditional sequencing method, NGS sequencers are now replacing Sanger in routine diagnosis. In particular, genomic variations of individuals (germline) or of cancerous tissues (somatic) can now be routinely analyzed for a number of medical applications ranging from genetic disease diagnostic to pharmacogenomics fine-tuning of medication in precision medicine practice. NGS consists in processing multiple fragmented DNA sequence reads, typically short ones (less than 300 nucleotide base pairs). The resulting reads can then be compared to a reference genome by means of a number of bioinformatics methods, to identify specific subsequences carrying small variants such as Single Nucleotide Polymorphisms (SNP) corresponding to a single nucleotide substitution, as well as short insertions and deletions (INDEL) of nucleotides in the DNA sequence compared to its reference.

Next-generation sequencing workflow automation and optimization

Next Generation Sequencing (NGS) enables in particular to detect and report small changes in the DNA sequence, such as single nucleotide polymorphisms (SNPs), insertions or deletions (INDELs), as compared to the reference genome, through bioinformatics methods such as sequencing read alignment, variant calling, and variant annotation. NGS workflows refer to the configuration and combination of such methods into an end-to-end genomic analysis application. In genomic research practice, NGS workflows are often manually setup and optimized using for instance dedicated scripts on a UNIX operating system, dedicated platforms including a graphical pipeline representation such as the Galaxy project, and/or a combination thereof. As clinical practice develops, NGS workflows may no longer be experimentally setup on a case-per-case basis, but rather integrated in SaaS (Software as a Service), PaaS (Platform as a Service) or IaaS (Infrastructure as a Service) offerings by third party providers. In that context, further automation of the NGS workflows is key to facilitate the routine integration of those services into the clinical practice.

While next generation sequencing methods have been shown more efficient than traditional Sanger sequencing in the detection of SNPs and INDELs, their specificity (rate of true positive detection for a given genomic variant) and sensitivity (rate of true negative exclusion for a given genomic variant) may still be further improved in clinical practice. The specificity and sensitivity of NGS genomic analysis may be affected by a number of factors:

- Biases introduced by the sequencing technology, for instance due to:

  ◦ Length of the reads relative to the length of the fragments;

  ◦ Too small number of reads (read depth);

  ◦ Errors or low quality bases introduced during sequencing;

  ◦ Inherent difficulties in counting homopolymer stretches, resulting in insertion and deletion errors;

- Biases introduced by the DNA enrichment technology, for instance due to:

∘ Primers or probes non-specific binding, for instance due to storing the assay at a low temperature for too long, or due to too small amount of DNA in the sample;

∘ Introduction of sequence errors caused by imperfect PCR amplification and cycling, for instance due to temperature changes;

∘ Suboptimal design of the probes or primers. For example, mutations may fall within the regions of the probes or primers;

∘ Enrichment method limitations. For instance, long deletion may span the amplified region;

∘ Cross-contamination of data sets, read loss and decreased read quality due to fragment tagging with barcodes, adapters and various pre-defined sequence tags;

∘ Chimeric reads in long-insert pair-ended reading.

• Biases introduced by the sample itself, for instance due to:

∘ Somatic features, in particular in cancer diagnosis based on tumor sample sequencing;

∘ The type of biological sample, e.g. FFPE, blood, urine, saliva, and associated sample preparation issues, for instance causing degradation of DNA, contamination with alien DNA, or too low DNA input.

• Biases introduced by the genomic data structure of certain regions specifically, for instance due to:

∘ High ratio of GC content in the region of interest;

∘ Presence of homopolymers and/or heteropolymers, that is partial genomic sequence repetitions of one or more nucleotides in certain regions, causing ambiguities in initial alignment, and possibly inherent sequencing errors;

∘ Presence of homologous and low-complexity regions;

∘ Presence of non-functional pseudogenes that may be confused with functional genes, in particular in high-repeat genomic regions of the human genome when the DNA fragments are not long enough compared to the read length.

This limits the efficient deployment of NGS in routine genomic analysis applications, as a different genomic data analysis workflow need to be manually organized and configured with different sets of parameters by highly specialized personnel for each application to meet the clinical expectations in terms of specificity and sensitivity. The automation of genomic data processing workflows is particularly challenging as the workflows need to be adapted to the specific data biases introduced by the upstream NGS biological processes on the one hand and the genomic data structures inherent to the current application on the other hand. In early deployment of genomic testing, a limited number of tests and setups were processed by dedicated platforms, which could be manually setup, configured and maintained by highly skilled specialized staff. This approach is costly and does not scale well as more and more tests have to be conducted in daily operation by a single multi-purpose genomic analysis platform.

Next-generation sequencing analysis of homopolymer and heteropolymer variants

In terms of automating the NGS analysis, special attention needs to be devoted to the inherent difficulties in characterizing the indel variants in homopolymer and/or heteropolymer regions of the reference human genome. Mischaracterization of some homopolymer or heteropolymer variants may result in false positive detection of certain traits and diseases in a diversity of diagnosis applications. A statistical method to better detect insertion and deletion in a homopolymer region and detecting the corresponding heterozygosity is described in US patent application 2014/0052381 by Utiramerur et al. They observed that in an NGS genomic analyzer workflow, the read alignment is not necessarily correct, but it may be possible to determine the heterozygosity from the distribution of base calling residuals based on measured and model-predicted values in the homopolymer regions by using a Bayesian peak detection approach and best-fit model, as homozygous regions tend to have a unimodal distribution while heterozygous regions tend to have a bimodal distribution. From the best-fit model, it is also possible to derive the homopolymer length value for both alleles in the homozygous (unimodal distribution) case, or two different homopolymer length values, one for each allele, in the heterozygous case (bimodal distribution). While this method may facilitate the identification of the length of short homopolymer regions as the associated flow space densities clearly exhibit a peak value, we observed that it is significantly more difficult to classify longer homopolymers as well as heteropolymers.

Pending patent application PCT/EP2019/065777 by Lin and Zu describes a genomic data analyzer configured to detect and characterize, with a variant calling module, genomic variants from next generation sequencing reads out of a pool of enriched genomic patient samples in repeat patterns regions of the human genome such as homopolymers or heteropolymers. The variant calling module may estimate the probability distribution of the length of the repeat pattern for each patient sample and cross-analyze it against other samples in a single experimental pool to identify best-fit variant models for each pair of samples. The variant calling module may further group samples according to their matching best-fit variant models and identify which group of patient samples carries the wild type reference without the need for control data in the pool. The variant calling module may subsequently characterize the homozygous or heterozygous repeat patterns variants for each patient sample with improved specificity and accuracy even in the presence of next generation sequencing biases. This genomic data analyzer is however primarily suited to the case of variants for which a well characterized wild type reference (with constant length) can be assumed in the best-fit variant models.

Next-generation sequencing Microsatellite status detection with NGS

In the human genome, microsatellite sequences are commonly found as homopolymer (mononucleotide) or heteropolymer (several nucleotides) sequences repeated 5 to 50 times in the DNA sequences. More than 500000 microsatellite loci have been found in the human genome, in coding as well as non-coding regions, corresponding to near 3% of the human DNA. It is estimated that at least 100000 to 150000 of these loci are highly variable in the human population germline DNA. Over 20 unstable microsatellite repeat loci have been identified as the cause of dozens of neurological diseases in human. Moreover, in somatic DNA mutations and in particular in tumor cells, microsatellite instability (MSI) is regularly observed as a genomic alteration due to insertions or deletions of a few nucleotides in the microsatellite repeat regions based upon one nucleotide repeat (homopolymers) or a few nucleotides (heteropolymers), due a DNA mismatch repair system deficiency. Thus, in a number of cancers, and in particular in uterine, colon and stomach cancers such as UCES (Uterine Corpus Endometrial Carcinoma), COAD (Colon Adenocarcinoma) and STAD (Stomach adenocarcinoma), a few microsatellite loci are routinely used as biomarkers in diagnosis and prognosis, for instance with the Bethesda panel, the Hamelin panel or the Promega test kit for Lynch syndrome. Most commonly tested MSI biomarkers genomic loci are homopolymers of at least 20bp such as BAT-25, a 25-repeat poly(T) tract located within intron 16 of the c-kit oncogene, which is typically found as a shorter version in tumor DNA; BAT-26, a 26-repeat poly(A) tract located within the fifth intron of the MSH2 gene, monomorphic at a length of 26 bp in 99% of individuals of ethnic European ancestry, yet polymorphic at a length of 15, 20, 22 or 23 bp in up to 25% of individuals of ethnic African ancestry, which is also typically found as a shorter version in tumor DNA; as well as NR-21, NR-22, NR-24, MONO-27, BAT-40, and/or CAT25. More recently, MSI status has also been used to identify the most relevant personalized medicine treatment based on immunotherapy, for instance with pembrolizumab, and with immune checkpoint blockade therapy in solid tumors.

More generally, determining MSI status may be of interest to facilitate the diagnosis, choice of treatment and prognosis in a diversity of cancers such as colorectal adenocarcinoma, endometrial cancer, bladder cancer, breast carcinoma, cervical cancer, cholangiocarcinoma, esophageal and esophagogastric junction carcinoma, extrahepatic bile duct adenocarcinoma, gastric adenocarcinoma, gastrointestinal stromal tumors, glioblastoma, liver hepatocellular carcinoma, lymphoma, malignant solitary fibrous tumor of the pleura, melanoma, neuroendocrine tumors, NSCLC, female genital tract malignancy, ovarian surface epithelial carcinomas, pancreatic adenocarcinoma, prostatic adenocarcinoma, small intestinal malignancies, soft tissue tumors, thyroid carcinoma, uterine sarcoma, uveal melanoma, and any combination thereof.

Prior art MSI tests are primarily based upon PCR amplification of DNA fragments from tumor tissue samples, followed by capillary electrophoresis or melting curve analysis to measure the fragment length polymorphisms, and compared to the germline measurement from a blood sample to characterize the instability of each MSI loci length in the tumor test set relative to the patient germline MSI lengths. With those prior art non-NGS based panels, patients can be categorized as:

- Micro-satellite stable, MSS: no evidence of any of the biomarker loci exhibiting instability.
- Micro-satellite instable-low, MSI-L: evidence of instability in only one marker locus.
- Microsatellite instable-high, MSI-H: evidence of instability in at least two marker loci.

These workflows do not scale well with the increasing medical demand for MSI characterization. In contrast, next-generation sequencing genomic analyzers facilitate the high throughput analysis of multiple genomic regions in multiplexed DNA samples, so that potentially more MSI loci can be used as biomarkers with improved limits of detection (LOD), in particular with the development of circulating tumor DNA (ctDNA) and cell free DNA (cfDNA) NGS analysis methods. Moreover, beyond the 5 or 7 standard MSI loci tested in clinical oncology practice since the mid 2000s, recent research has identified many more MSI loci frequently mutated in tumor DNA and thus potentially associated with cancer diagnosis, prognosis, and treatment. As the cost of high throughput sequencing keeps on decreasing, there is a growing interest in extending the prior genomic data analytics, also on microsatellite variants. WO2013/153130 by Lambrechts et al. from the VIB identifies a set of 106 frequent recurrently mutated genomic markers as homopolymers of 8 to 18 bp lengths (mostly in the 10-13bp range) associated with the MMR-deficient tumors, which may by treated with PARP inhibitors, but did not develop NGS experiments with these loci. WO2017/112738 by Perry et al. from Myriad Genetics identifies another set of 35 homopolymer microsatellite loci which can be used to identify the MSI status of a tumor sample, by detecting 20 to 33 indels in the 35 microsatellite regions relative to a known reference DNA sequence or the sequence of germline DNA from the tested patient. WO2018/037231 by Burn et al. from the University of Newcastle Upon Tyne discloses another set of 120 short markers which can discriminate between MSI-H and MSS status for colorectal cancer (CRC) and Lynch Syndrome diagnosis and chemotherapy treatment indication, for instance with pembrolizumab, irinotecan, bevacizumab, cisplatin, carboplatin, 5-fluorouracil (5-FU) and others. Burn et al. selected the markers specifically as short mononucleotide repeats of up to 12 nucleotides maximum, so that they are less likely to be polymorphic, and also explicitly excluded from their set those loci within this range which are known to be polymorphic in the human population. Thanks to the plausible exclusion of the polymorphic loci from the selected set of microsatellite sites, the patient tumor genomic data can be simply compared to the monomorphic wild type reference genome without the need to compare with the patient germline DNA as it would be required with polymorphic loci such as for instance BAT-26.

On the analytics side, the microsatellite regions remain particularly challenging to characterize with next-generation sequencing statistical bioinformatics methods because of the higher ratio of NGS biases in repeat regions, the low quality coverage signal in some NGS experiments, the low variant fraction in the input samples, and/or the MSI-specific fact that there is no strong germline wild type reference signal (with a predefined repeat loci length) to compare to for unbiasing purposes; indeed, many loci may be polymorphic and vary from patient to patient even in the germline cells.

As reviewed by Baudrin et al. in "Molecular and computational methods for the detection of microsatellite instability in cancer", Frontiers in Oncology, Dec 2018 (https://doi.org/10.3389/fonc.2018.00621), several bioinformatics NGS methods have recently been developed in combination with different library preparation protocols (wet lab protocols, assays or kits), including Whole Genome Sequencing (WGS), Whole Exome Sequencing (WES) and targeted gene sequencing (TGS) protocols, the latter being either amplicon-based or capture-based. Salipante et al. from the University of Washington proposed an NGS-based MSI detection method, called mSINGS, in "Microsatellite instability detection by next generation sequencing", Clinical Chemistry 60:9, pp. 1192-1199 (2014), presenting NGS testing results for 15 to 2957 microsatellite markers out of a targeted gene enrichment. Their method compares the patient microsatellite lengths to those of a reference population of MSI-negative individuals, and provides a quantitative assessment of the MSI status over multiple loci rather than a qualitative MSS/MSI-L/MSI-H ternary classification. The mSINGS method was later applied to a diversity of cancers by characterizing the MSI status across more than 200000 microsatellite loci, by using high throughput exome sequencing of tumor-normal tissue pairs, as part of the MOSAIC method described by Hause et al in "Classification and characterization of microsatellite instability across 18 cancer types", Nature Medicine 22, 1342-1350 (2016). Cortes-Ciriano et al. in "A molecular portrait of microsatellite instability across multiple cancers", Nature Communications also evidenced the applicability of analyzing a broader set of microsatellite loci acress the whole genome to characterize the MSI status in different cancers, using the comparisons of the distribuions of the repeat lengths from tumour and matched normal genomes at each locus using the Kolmogorov-Smimof statistics.

As reviewed by Baudrin et al., the prior art methods MSISensor, MANTIS, MSings, the Cortes-Ciriano method and MSI-ColonCore use a similar genomic analysis workflow, comprising the steps of:

   1. read alignment of NGS raw reads data from tumor samples
   2. microsatellite loci identification (from 15 loci up to more than 1000 microsatellite markers)
   3. indel/allele calling for each microsatellite locus
   4. for each locus, comparison of the length distribution in the tumor samples with the normal samples (baseline or paired reference depending on the genomic analysis tool) using statistical tests (Z-score, average distance, Kolomogorov-Smirnov or chi square depending on the genomic analysis tool)
   5. for each tumor sample, MSI calling based on thresholding for a minimum ratio of instable loci out of the scores or distances calculated in the former steps for most tools, or, specifically for the Cortes-Ciriano method, a random forest analysis for the MSI status classification.

The FDA-approved FoundationOne CDx NGS panel from Foundation Medicine uses a customized analysis workflow designed to detect various genomic alterations, including microsatellite instability in 95 undisclosed intronic homopolymer repeat loci of length 10 to 20 bp. For each of these 95 MSI loci, the repeat length distribution is calculated over all mapping reads, and the mean and the variance of the repeat length distribution is used in a 190-dimension data projection into the MSI score, a single value corresponding to the first component of a principal component analysis. The MSI-H or MSS status is assessed by manual unsupervised clustering rather than by an automated workflow, which does not scale well with the increasing demand for NGS analytics by worldwide laboratory and hospitals. Recent research by Foundation Medicine uses a similar approach, as reported for instance in "Real-time targeted genome profile analysis of pancreatic ductal adenocarcinomas identifies genetic alterations that might be targeted with existing drugs or used as biomarkers", gastrology 2019;156:2242-2253 by Singhi et al. which reports the genomic MSI characterization of PCAD tumors from FFPE patient samples according to the NGS analysis of a subset of 114 intronic homopolymer loci out of a set of 1897 potential microsatellite loci with adequate coverage on FFPE samples on their custom targeted NGS workflow. The 114 loci are filtered based on their hg19 reference repeat length which is chosen in the range of 10 to 20bp so that, according to the authors, they are long enough to produce a high rate of DNA polymerase slippage but short enough to facilitate alignment in their NGS workflow with 49bp read length. The measured mean and variance of the repeat length distributions of the NGS reads associated with each patient sample at each of the 114 loci are analyzed with principal component analysis to produce the MSI score, enabling to classify the tumor as MSS, MSI-ambiguous (but not necessarily MSI-L - the data is not discriminant enough for that classification), or MSI-H.

The FDA-approved MSK-Impact assay from the Memorial Sloan Kettering uses an NGS computational workflow with the MSIsensor tool from Niu et al. "MSIsensor: microsatellite instability detection using paired tumor-normal sequence data", Bioinformatics 30(7):1015-1066 (2014). By comparing the DNA coverage for tumor samples with a panel targeting 468 genes associated with cancer against the matched normal DNA information, the MSK-Impact assay enables to assess the number and length of over 1000 microsatellite homopolymer markers rather than the limited subset of 5 or 7 MSI loci used by the prior art non-NGS assays. The MSI loci are assumed somatic if the k-mer distributions are significantly different between the tumor and the matched normal genomic data as measured with a standard multiple testing correction of $\chi 2$ p-values. The tool calculates accordingly a continuous score rather than a discrete decision (MSS, MSI-L, MSI-H), and classifies the patient tumor as MSS if the score is below 10, as MSI-H otherwise. One major limitation of this assay, as well as of most prior art methods, is that it requires tumor/normal paired sequencing data; it cannot work with tumor-only data.

More recently, Niu at al. have announced an updated MSIsensor2 software working only with tumor sequencing data, indifferently from Cell-free DNA (cfDNA), Formalin Fixed Paraffin Embedded (FFPE) or other patient sample type, as announced at https://github.com/niu-lab/msisensor2 (retrieved on Nov 20, 2019). According to their benchmarks, the tool performs similarly to the MSIsensor software in terms of MSI classification, but it now runs 10 times faster on a targeted panel of 500 genes, as well as with WES or WGS data sets. The authors mention that the tool is based on a novel machine learning algorithm, but did not disclose the source code or computational method details. They only indicated that for use with a dedicated MSI set, for instance on specific genes, the tool requires a dedicated training to build the data model specifically for the selected MSI set. No constraints on the choice of the MSI loci has been disclosed by the authors, but, according to the github file updates on Oct 12, 2019, the early version of the software was using the same parametrization options as MSIsensor, assuming a default minimal homopolymer size of 5 and a maximal size of 50 base pairs, and a default minimal homopolymer size for distribution analysis of 10. With the MSIsensor tool, these parameters were configurable as command line options, while in the latest MSIsensor2 release, they are predefined to default values and may actually vary with the machine learning model, possibly throughout the whole MSI.

WO2019/108807 by Georgiadis et al. from Personal Genome Diagnostics describes a process for reporting an MSI status from a cell-free DNA sample of the patient blood or plasma in order to determine whether the patient cancer is suitable for immune checkpoint inhibitor therapy comprising an antibody such as anti-PD-1, anti-IDO, anti-CTLA-4, anti-PD-LI or anti-LAG-3. The process comprises comparing how the peaks of the length distribution of the measured repeat tract deviate from peaks of a reference distribution from a matched normal DNA sample corresponding to the healthy model, after some barcoding error correction steps to facilitate the digital peak finding (DPF). The proposed DPF method retains only the clearly discriminating local peaks with sufficient coverage once filtered based on several simple local heuristics. The method operates either on standard MSI subsets of 5 loci (BAT25, BAT26, MONO27, NR21, NR24) or 7 loci (BAT-25, BAT-26, MONO-27, NR-21, NR-24 homopolymers, and PentaC and PentaD heteropolymers), or with an additional set of 65 undisclosed microsatellite regions. Samples are classified as MSI-H if > 20% of loci were MSI. This process assumes the availability of a reference distribution which may be from a healthy patient DNA or from a human genome reference or any other suitable source, but the document does not disclose how to build such a suitable model including possibly polymorphic microsatellite regions for which there may not be any clearly discriminating peak in the length distribution data.

There is therefore a need for improved high throughput genomic analysis methods to characterize the MSI status of patient samples that jointly meet the following requirements: the methods should be suitable for an automated NGS workflow not requiring manual classification, and fast enough to be deployed at a large scale in current computational architectures; the methods should provide comparable sensitivity and specificity compared to the current clinical practice MSI testing protocols and assays, while being indifferently applicable to various sets of microsatellite loci in accordance with the actual clinical application needs; the methods should not rely upon somatic-normal pair matching with a germline sample; still, the methods should be suitable for detection even with low somatic variant fraction below 10% of the sample, for instance out of FFPE samples or liquid biopsy samples.

Summary

A method is accordingly proposed for determining a microsatellite instability (MSI) status of a patient comprising: obtaining DNA fragments from a biological DNA sample from a patient, the sample comprising cells from a solid tissue or a bodily fluid; sequencing the DNA fragments with a high throughput sequencing technology to obtain a plurality of data reads for each DNA fragment; aligning the data reads to a reference genome DNA sequence comprising a predefined set of N microsatellite genomic loci; at each microsatellite genomic locus $i$ in the predefined set of microsatellite genomic loci, measuring a patient sample distribution $D_{MSI}$ of the nucleotide repeat lengths for the set of aligned data reads mapped to the reference genome DNA sequence at the microsatellite locus $i$ and estimating a local MSI status score $s_i$ as a function of the difference of the measured patient sample distribution $D_{MSI}$ of the nucleotide repeat lengths relative to a reference background distribution model $D_{MSS}$ of the nucleotide repeat lengths at the microsatellite genomic locus $i$; determining a global MSI score $S$ to characterize the MSI status of the patient sample as a function of all estimated local MSI status scores $s_i$, $i$=1 to N, each microsatellite genomic locus in the predefined set being a mono-homopolymer repeat of a single nucleotide and having a reference repeat length of at least 13 nucleotides (13bp) and at most 25 nucleotides (25bp).

In a possible embodiment, the local MSI status score $s_i$ may be calculated as a function of at least two independent scalar parameters $p_1$, $p_2$ of a multi-parametric function $F(D_{MSS}, p_1, p_2)$ of the reference background distribution model $D_{MSS}$ of the nucleotide repeat length such that $D_{MSI} = F(D_{MSS}, p_1, p_2)$ at the microsatellite genomic locus $i$.

In a further possible embodiment, the local MSI status score $s_i$ may be calculated as a function of three independent scalar parameters $p_1$, $p_2$, $p_3$ of a multi-parametric function $F(D_{MSS}, p_1, p_2, p_3)$ of the reference background distribution model $D_{MSS}$ of the nucleotide repeat length such that $D_{MSI} = F(D_{MSS}, p_1, p_2, p_3)$ at the microsatellite genomic locus $i$.

The independent scalar parameters may be the variant fraction $p_1$ measuring the ratio of somatic DNA content relative to the total DNA content in the patient sample, a microsatellite length shift value p2 characterizing by how many insertions or deletions the somatic microsatellite length has shifted in the somatic DNA normalized relative to the microsatellite stable status length at the microsatellite genomic locus $I$, and possibly a microsatellite length stability value p3 characterizing how variable the somatic microsatellite length shift is in the somatic DNA content.

The independent scalar parameters p={$p_1$, $p_2$} or p={$p_1$, $p_2$, $p_3$} at locus $i$ may be inferred by minimizing the difference between the measured patient distribution $D_{MSI}$ and the predicted patient distribution $F(D_{MSS}, p)$ at locus $i$.

The local MSI status score may be estimated as a function of at least two of the independent scalar parameters at each locus $i$, and the global MSI score may be calculated as the sum of the local MSI status scores over the N loci, or a normalized MSI score calculated as the sum of the local MSI status scores normalized to the highest local MSI score over the N loci, and/or an MSI score count calculated as the number of loci in the set of N loci where the local MSI score is over a predefined threshold.

The microsatellite instability (MSI) status of the patient may be determined as a positive status if the global MSI score $S$ over the N loci is above a predefined cutoff value, and as a negative status if the global MSI score $S$ over the N loci is below the predefined cutoff value.

Brief Description of the Drawings

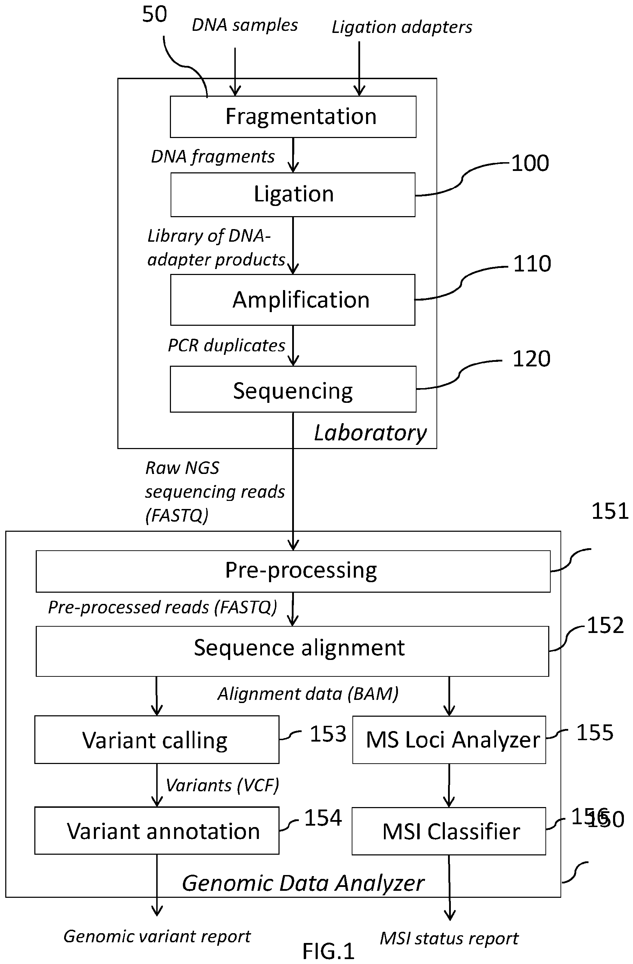

FIG. 1 is a schematic representation of a genomic analysis workflow comprising a laboratory process (also known as the "wet lab" process) and a bioinformatics workflow (also known as the "dry lab" process).

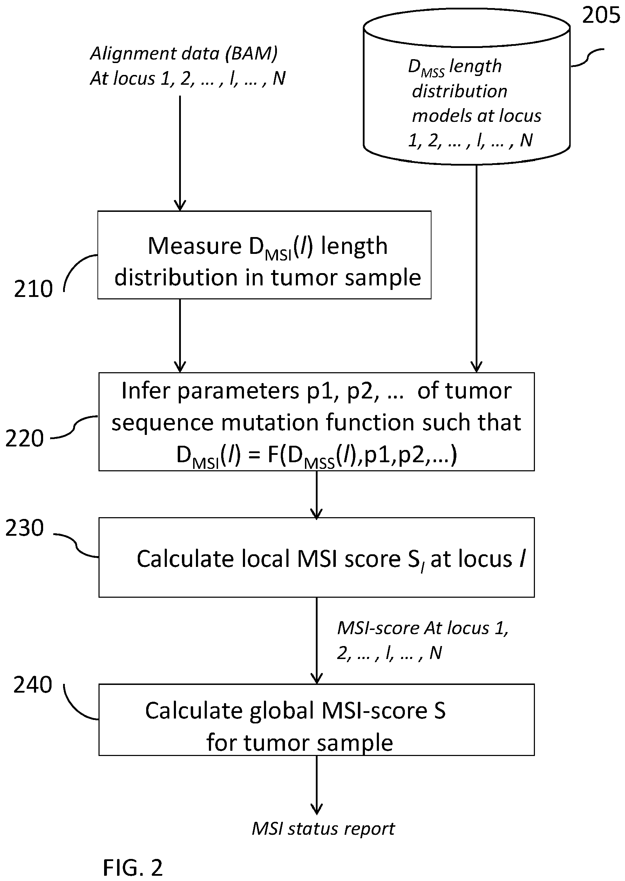

FIG. 2 is a schematic representation of an exemplary MSI analysis computational workflow according to some embodiments of the present disclosure.

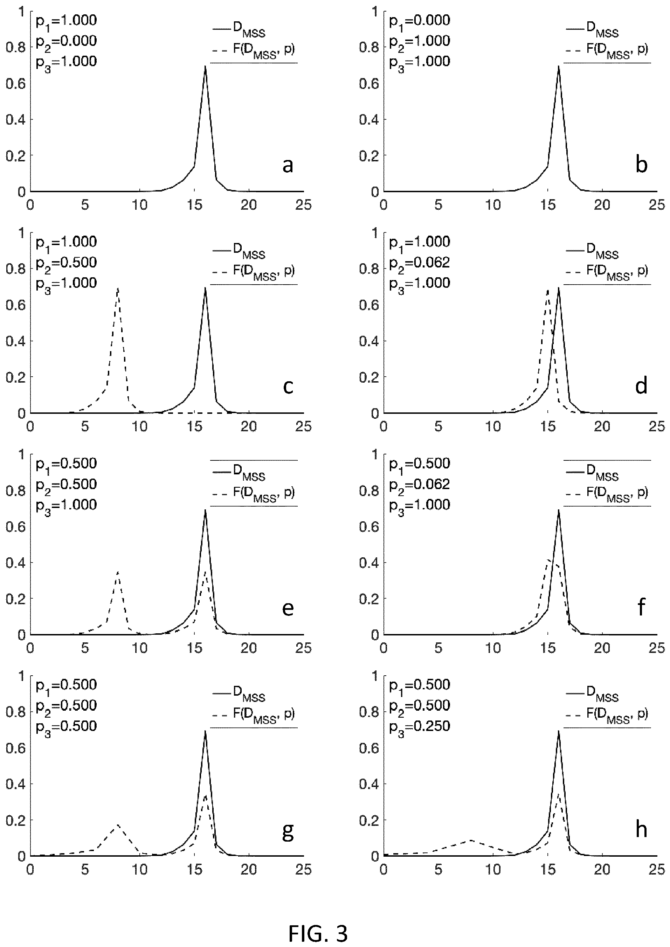

FIG. 3 illustrates the theoretical transformation of the length distribution of a background stable model into different possible MSI lengths distribution according to different variables, namely the variant fraction, the average somatic MSI length shift relative to a reference background stable model distribution length peak and the variability of the MSI length into samples comprising different variant fractions of somatic DNA over total DNA.

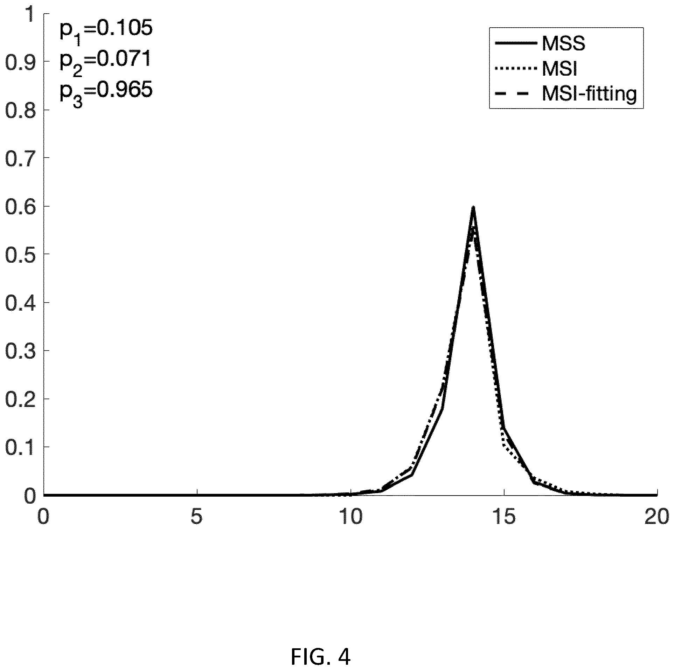

FIG. 4, FIG. 5 and FIG. 6 represent the microsatellite repeat lengths distributions for a background stable model, a measured patient sample, and the fitted MSI repeat length distribution and its inferred parametrization according to the proposed methods at three different microsatellite loci, to highlight the advantages of the proposed methods over the prior art peak-finding and curve-fitting methods to more accurately determine the MSI-status of the patient sample.

Detailed Description

The particulars shown herein are by way of example and for purposes of illustrative discussion of the various embodiments only and are presented in the cause of providing what is believed to be the most useful and readily understood description of the principles and conceptual aspects of the methods and compositions described herein. In this regard, no attempt is made to show more detail than is necessary for a fundamental understanding, the description making apparent to those skilled in the art how the several forms may be embodied in practice.

The proposed methods and systems will now be described by reference to more detailed embodiments. The proposed methods and systems may, however, be embodied in different forms and should not be construed as limited to the embodiments set forth herein. Rather, these embodiments are provided so that this disclosure will be thorough and complete, and will fully convey the scope to those skilled in the art.

Unless otherwise defined, all technical and scientific terms used herein have the same meaning as commonly understood by one of ordinary skill in the art to which this invention belongs. The terminology used in the description herein is for describing particular embodiments only and is not intended to be limiting. As used in the description and the appended claims, the singular forms "a," "an," and "the" are intended to include the plural forms as well, unless the context clearly indicates otherwise.

Unless indicated to the contrary, the numerical parameters set forth in the following specification and attached claims are approximations that may vary depending upon the desired properties sought to be obtained and thus may be modified by the term "about". At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

*Definitions*

A **"DNA sample"** refers to a nucleic acid sample derived from an organism, as may be extracted for instance from a body tissue or fluid. The organism may be a human, an animal, a plant, fungi, or a microorganism. The nucleic acids may be found in limited quantity or low concentration, such as fetal circulating DNA (cfDNA) or circulating tumor DNA in blood or plasma. A DNA sample also applies herein to describe RNA samples that were reverse-transcribed and converted to cDNA.

A **"DNA fragment"** refers to a short piece of DNA resulting from the fragmentation of high molecular weight DNA. Fragmentation may have occurred naturally in the sample organism, or may have been produced artificially from a DNA fragmenting method applied to a DNA sample, for instance by mechanical shearing, sonification, enzymatic fragmentation and other methods. After fragmentation, the DNA pieces may be end repaired to ensure that each molecule possesses blunt ends. To improve ligation efficiency, an adenine may be added to each of the 3' blunt ends of the fragmented DNA, enabling DNA fragments to be ligated to adaptors with complementary dT-overhangs.

A **"DNA product"** refers to an engineered piece of DNA resulting from manipulating, extending, ligating, duplicating, amplifying, copying, editing and/or cutting a DNA fragment to adapt it to a next-generation sequencing workflow.

A **"DNA-adaptor product"** refers to a DNA product resulting from ligating a DNA fragment with a DNA adaptor to adapt it to a next-generation sequencing workflow.

A **"DNA library"** refers to a collection of DNA products or DNA-adaptor products to adapt DNA fragments for compatibility with a next-generation sequencing workflow.

A **"Microsatellite"** refers to the multiple, continuous repetition of nucleotide patterns of one to nine base pairs, typically 5 to 50 times, in a genomic sequence. The human genome hosts hundreds of thousands of microsatellite loci, and microsatellites are prone to indel mutations (nucleotide insertions, deletions and combination thereof). Out of those, polymorphic microsatellites are microsatellites found with a high variability among a human population, with typically more than 1% heterozygosity for the microsatellite repeat length found in healthy individuals germline data.

"**MSI**" refers to a genomic microsatellite instability, a condition is which the length of one or more microsatellite repeat is shortened, possibly due to a deficiency in the MMR (DNA mismatch repair) pathway. MSI has been associated with a number of cancers, such as, but not limited to, colorectal, gastric and endometrial cancers. In the existing MSI testing panels, MSI-H characterizes that at least two MSI loci have been found in the sample, while MSI-L characterizes that solely one has been found (usually out of 5 or 7 microsatellite markers).

"**MSS**" refers to the stable status of a microsatellite.

A "**pool**" refers to multiple DNA samples (for instance, 48 samples, 96 samples, or more) derived from the same or different organisms, as may be multiplexed into a single high-throughput sequencing analysis. Each sample may be identified in the pool by a unique sample barcode.

A "**nucleotide sequence**" or a "**polynucleotide sequence**" refers to any polymer or oligomer of nucleotides such as cytosine (represented by the C letter in the sequence string), thymine (represented by the T letter in the sequence string), adenine (represented by the A letter in the sequence string), guanine (represented by the G letter in the sequence string) and uracil (represented by the U letter in the sequence string). It may be DNA or RNA, or a combination thereof. It may be found permanently or temporarily in a single-stranded or a double-stranded shape. Unless otherwise indicated, nucleic acids sequences are written left to right in 5' to 3' orientation.

A "**primer sequence**" refers to a nucleotide sequence of at least 20 nucleotides in length comprising a region of complementarity to a target DNA a part or all of which is to be elongated or amplified.

"**Ligation**" refers to the joining of separate double stranded DNA sequences. The latter DNA molecules may be blunt ended or may have compatible overhangs to facilitate their ligation. Ligation may be produced by various methods, for instance using a ligase enzyme, performing chemical ligation, and other methods.

"**Amplification**" refers to a polynucleotide amplification reaction to produce multiple polynucleotide sequences replicated from one or more parent sequences. Amplification may be produced by various methods, for instance a polymerase chain reaction (PCR), a linear polymerase chain reaction, a nucleic acid sequence-based amplification, rolling circle amplification, and other methods.

"**Sequencing**" refers to reading a sequence of nucleotides as a string. High throughput sequencing (HTS) or next-generation-sequencing (NGS) refers to real time sequencing of multiple sequences in parallel, typically between 50 and a few thousand base pairs. Exemplary NGS technologies include those from Illumina, Ion Torrent Systems, Oxford Nanopore Technologies, Complete Genomics, Pacific Biosciences, and others. Depending on the actual technology, NGS sequencing may require sample preparation with sequencing adaptors or primers to facilitate further sequencing steps, as well as amplification steps so that multiple instances of a single parent molecule are sequenced, for instance with PCR amplification prior to delivery to flow cell in the case of sequencing by synthesis.

An "**adapter**" or "**adaptor**" refers to a short double-stranded or partially double-stranded DNA molecule of around 10 to 100 nucleotides (base pairs) which has been designed to be ligated to a DNA fragment. An adaptor may have blunt ends, sticky ends as a 3' or a 5' overhang, or a combination thereof. For example, to improve ligation efficiency, an adenine may be added to each of the 3' blunt ends of the fragmented DNA prior to adaptor ligation, and the adaptor may have a thymidine overhang on the 3' end to base-pair with the adenine added to the 3' end of the fragmented DNA. The adaptor may have a phosphorothioate bond before the terminal thymidine on the 3' end to prevent an exonuclease from trimming the thymidine, thus creating a blunt end when the end of the adaptor being ligated is double-stranded.

A "**PCR duplicate**" refers to a copy generated by PCR amplification from a single stranded DNA molecule belonging to a DNA-adaptor product derived from an original DNA fragment.

A "**molecular tag**" or "**molecular barcode**" or "**molecular code**" or "**molecular identifier**" refers to a molecular arrangement such as a nucleic acid sequence which is fully and uniquely specified by its string of nucleotides.

"**Read trimming**" or "**Read pre-processing**" refers, in a bioinformatics workflow, to the filtering out, in the sequencing reads, of a set of nucleotides at the start of the read sequence string, such as for instance the nucleotides corresponding to the adaptor sequences, to extract the real DNA fragment sequence to be analyzed.

"**Aligning**" or "**alignment**" or "**aligner**" refers to mapping and aligning base-by-base, in a bioinformatics workflow, the pre-processed sequencing reads to a reference genome sequence, depending on the application. For instance, in a targeted enrichment application where the sequencing reads are expected to map to a specific targeted genomic region in accordance with the hybrid capture probes used in the experimental amplification process, the alignment may be specifically searched relative to the corresponding sequence, defined by genomic coordinates such as the chromosome number, the start position and the end position in a reference genome.

"**Variant calling**" or "**variant caller**" or "**variant call**" refers to identifying, in the bioinformatics workflow, actual variants in the aligned reads. Variants may include single nucleotide permutations (SNPs), insertions or deletions (INDELs), copy number variants (CNVs), as well as large rearrangements, substitutions, duplications, translocations, and others. Preferably variant calling is robust enough to sort out the real variants from the amplification and sequencing noise artefacts.

"**Consensus sequencing**" refers, in a bioinformatics workflow, to grouping sequencing reads into families of reads issued from the same double-stranded DNA fragment and/or the same DNA fragment strand, comparing them to detect errors due to the amplification and/or sequencing steps, and correcting the errors to produce a unique, deterministic consensus sequence for the double-stranded DNA fragment or the DNA fragment strand. Variant calling is then performed by processing the resulting consensus sequences, rather than the totality of reads.

**"Probabilistic sequencing"** refers, in a bioinformatics workflow, to grouping sequencing reads into families of reads issued from the same double-stranded DNA fragment and/or the same DNA fragment strand and performing variant calling directly on this data, by processing the totality of reads from different families in order to compute the probability of data supporting all the possible genotypes at each genomic position to be analyzed, by comparing the data with a probabilistic model.

*Genomic Analysis Workflow*

The methods disclosed may be integrated indifferently into a diversity of NGS genomic data analysis wetlab workflow systems. The tumor sample DNA may be extracted from a formalin fixed paraffin embedded (FFPE) sample or from a bodily fluid. The tumor sample DNA may be assayed for NGS genomic data analysis with a capture-based or an amplicon-based technology according to the assay provider protocols. The tumor sample DNA may then be analyzed with a NGS technology workflow such as a whole genome sequencing (WGS), whole exome sequencing (WES) or targeted enrichment technology to sequence at least a specific subset of genomic regions associated with cancer diagnosis or prognosis may be applied.

Such a genomic analysis workflow suitable for characterizing the microsatellite alteration status in genomic tumor samples possibly from low frequency DNA out of liquid biopsies is described with further detail with reference to FIG. 1. As will be apparent to those skilled in the art of DNA analysis, such a workflow comprises preliminary experimental steps to be conducted in a laboratory (also known as the "wet lab") to produce DNA analysis data, such as raw sequencing reads in a next-generation sequencing workflow, as well as subsequent data processing steps to be conducted on the DNA analysis data to further identify information of interest to the end users, such as the detailed identification of DNA variants and related annotations, with a bioinformatics system (also known as the "dry lab"). Depending on the actual application, laboratory setup and bioinformatics platforms, various embodiments of a DNA analysis workflow are possible. FIG. 1 describes an example of a workflow comprising a wet lab process wherein DNA samples are first fragmented with a fragmentation protocol 50 (optional) to produce DNA fragments. The DNA ends of these DNA fragments are then repaired and modified such as to be compatible with the adaptors that will be used. Adaptors as will be further described in more detail throughout this enclosure may then be joined by ligation 100 to the DNA fragments in a reaction mixture, so as to produce a library of DNA-adaptor products, in accordance with some of the proposed methods. The DNA library further undergoes amplification 110 and sequencing 120. In a next generation sequencing workflow, the resulting DNA analysis data may be produced as a data file of raw sequencing reads in the FASTQ format. The workflow may then further comprise a dry lab Genomic Data Analyzer system 150 which takes into input the raw sequencing reads for a pool of DNA samples prepared with the ligation adaptors according to the proposed methods, and applies a series of data processing steps to identify genomic variants, for instance as a genomic variant report for the end user. An exemplary Genomic Data Analyzer system 150 is the Sophia Data Driven Medicine platform (Sophia DDM) as already used by more than 1000 hospitals worldwide in 2019, but other systems may be used as well. Different detailed possible embodiments of data processing steps as may be applied by the Genomic Data Analyzer system 150 are described for instance in the international PCT patent application WO2017/220508, but other embodiments are also possible.

In a preferred embodiment, the Genomic Data Analyzer system 150 may first apply one or more pre-processing steps 151 to produce pre-processed reads from the raw sequencing reads inputs. The pre-processing steps may for instance comprise adaptor trimming, as well as read sorting, to analyze and group reads in families of reads issued from similar DNA fragments in accordance with the proposed adaptor ligation methods and numerical coding methods as will be further described herein. In a possible embodiment, the raw reads as well as the pre-processed reads may be stored in the FASTQ file format, but other embodiments are also possible.

The Genomic Data Analyzer system 150 may further apply sequence alignment 152 to the pre-processed reads to produce read alignment data. In one embodiment, the read alignment data may be produced for instance in the BAM or SAM file format, but other embodiments are also possible.

The Genomic Data Analyzer system 150 may further apply variant calling 153 to the read alignment data to produce variant calling data. In one embodiment, the variant calling data may be produced for instance in the VCF file format, but other embodiments are also possible.

The Genomic Data Analyzer system 150 may further apply variant annotation 154 to the read alignment data to produce a genomic variant report for each DNA sample. In one embodiment, the genomic variant report may be visualized by the end user on a graphical user interface. In another possible embodiment, the genomic variant report may be produced as a text file for further data processing. Other embodiments are also possible.

In a preferred embodiment, the Genomic Data Analyzer system 150 may comprise an MS loci analyzer 155 to analyze the distributions of microsatellite biomarker loci lengths from the read alignment data. The Genomic Data Analyzer system 150 may further comprise an MSI classifier 156 to produce an MSI status report for each DNA sample according to the classification of the analyzed microsatellite biomarker loci lengths distributions from the MS loci analyzer. In one embodiment, the MSI status report may be visualized by the end user on a graphical user interface. In another possible embodiment, the MSI status report may be produced as a text file for further data processing or communication. Other embodiments are also possible.

*Microsatellite loci*

The present disclosure provides for the detection of MSI out from a subset of microsatellite loci in the human genome selected as homopolymers with a reference length in the human genome of at least 13bp and at most 25bp.

The microsatellite loci may be assayed from a body tissue sample, a liquid biopsy, a blood sample or a plasma sample. In the case of cancer diagnosis, the microsatellite loci may be assayed from cell-free DNA (cfDNA) or circulating tumor DNA (ctDNA) from a liquid biopsy, a blood sample or a plasma sample, or from a Formalin Fixed Paraffin Embedded (FFPE) tumor tissue sample for each patient. The microsatellite loci may be located in coding regions, non-coding regions, intronic regions, exons, or a combination thereof. The microsatellite loci may be analyzed from a tumor sample such as a with whole genome sequencing data (WGS), whole exome sequencing data (WES), amplicon-based targeted enrichment probes sequencing data, capture-based targeted enrichment sequencing data with various barcoding and molecular identification tagging technologies, such as single strand sequencing, double strand sequencing, duplex sequencing, circular sequencing, variable length tagging sequencing, and other methods known by those skilled in the art of NGS wet lab practice. Various high throughput sequencing technologies may be used, including those from Illumina, Ion Torrent Systems, Oxford Nanopore Technologies, Complete Genomics, Pacific Biosciences, and others, to produce a plurality of NGS reads of a predefined size (for instance, 50pb, 100bp, 150 bp, 200bp, 250bp, 300bp and beyond) for each DNA fragment assayed from the input sample.

In a possible embodiment, the subset of microsatellite loci may be selected from the best performing microsatellite markers in MSI diagnosis for one or more cancers as reported in prior art work, for instance by Salipante in the mSINGS experiments or by Cortes-Ciriano. In a possible embodiment, the subset if microsatellite loci may be selected as the 10, 20, 30, 40, 50, 60, 70, 80, 90, 100, 110, 120, 130, 140, 150, 200, 250 best performing MSI homopolymer markers with a reference repetition length of at least 13bp and at most 25bp.

*Background model and related parameters*

Due to the homopolymer repeats, the NGS measurements tend to be noisy, so even in a healthy individual germline sample measurement, the measured homopolymer length at any microsatellite locus usually follows a variable length distribution instead of a constant number. Furthermore, this variable microsatellite length distribution may not exhibit a clear, unique peak of all read counts centered at the human genome reference microsatellite length. The genomic analyzer 155 may rather estimate a microsatellite length distribution, corresponding to the stable status in a given population: $D_{MSS}$ for each microsatellite locus $i$. If the locus is monomorphic in the reference population, the $D_{MSS}$ distribution can be assumed to match a monomodal reference model. If the locus is polymorphic in the reference population, the $D_{MSS}$ distribution may be assumed to match a multimodal model. There is therefore a different $D_{MSS}$ stable reference length distribution at each microsatellite locus $i$, $1<=i<=N$, where N is the total number of MSI loci to test. Preferably, each $D_{MSS}$ distribution may be represented by a vector of read count values for each possible homopolymer length $l$ at the microsatellite locus $i$, normalized relative to the total number of reads used in the length measurement at the microsatellite locus $i$. Other embodiments are also possible, for instance by measuring the relative abundance of individual length values at a locus relative to the read count of the most frequently occurring length value corresponding to the normalization reference value of 1, as proposed for instance in the MOSAIC method from Hause.

In somatic cells, the mutated DNA may include indels characterizing the MSI status at one or more microsatellite loci. For patient samples with an MSI inducing a somatic variant homopolymer length different from the MSS reference homopolymer length at a microsatellite locus $i$ (for instance shortened or enlarged by 1bp, 2bp, 3bp, etc), the genomic analysis may thus measure 210 a microsatellite length distribution $D_{MSI}$. Preferably, each measured $D_{MSI}$ may be represented by a vector of read count values for each possible homopolymer length $l$ at the microsatellite locus $i$, normalized relative to the total number of reads used in the length measurement at the microsatellite locus $i$. If the sample has no microsatellite instability at locus $i$, theoretically the measured somatic $D_{MSI}$ is the same as the stable background reference model $D_{MSS}$. Conversely, if the sample has a microsatellite instability at locus $i$, the measured somatic $D_{MSI}$ differs from the background reference model $D_{MSS}$ (by a shift $p$ of 1bp, 2bp, 3bp, etc). It is therefore possible to estimate the patient sample MSI status at any given loci by comparing $D_{MSI}$ to $D_{MSS}$, for instance by measuring a distance metrics between them.

As will be apparent to those skilled in the art, the prior art methods mentioned above achieve a direct statistical comparison between the $D_{MSI}$ and $D_{MSS}$, without taking into account that multiple independent parameters may contribute to their difference. We propose to better characterize the underlying biological principles of an MSI event, by taking into account what is happening in the genome, during the transformation from normal cells to somatic cells. The closer the genomic analysis model matches the biological events, the better the MSI characterization performance. To achieve this, for each sample, at each microsatellite locus $i$, the Genomic Data Analyzer 150 may infer a function F of at least two independent scalar parameters $p_1$, $p_2$ that can transform the background MSS distribution($D_{MSS}$) into the measured MSI distribution($D_{MSI}$) at that locus in the sample genomic data:

$$F(D_{MSS}, p_1, p_2, ...) = D_{MSI}$$

To capture as closely as possible the underlying biological principles of MSI events, each parameter needs to be carefully designed. Moreover, the number of parameters needs to be carefully selected: with less parameters, it may not be possible to represent the biological principle variants behind the data, while more parameters may increase the risk of overfitting. In our experiments over two hundred microsatellite biomarker loci chosen from the literature (Salipante, Cortes-Ciriano), a model with 3 independent scalar parameters $p_1$, $p_2$, $p_3$ for the transform function provides a good enough fit. However, based on the data type, the definitions of the parameters and the number of parameters can be variable.

First of all, we experimented that prior art MSI detection approaches suffer from a number of limitations when applied to automatized genomic data analysis workflows from different laboratories possibly with different biological samples. Indeed, in practice, patient samples usually comprise a mixture of normal cells with germline DNA and somatic cells with mutated DNA, so the measured microsatellite length distribution $D_{MSI}$ at any microsatellite locus $i$ is partly due to the somatic DNA microsatellite homopolymer length contribution, partly due to the germline DNA microsatellite homopolymer length contribution. The variant fraction corresponding to the ratio $p_1$ of somatic DNA in the patient sample may be variable per patient and may be relatively low in the case of cfDNA or ctDNA measurements. This complicates the evaluation of the actual length distribution characterizing the patient sample as the lower the somatic ratio $p_1$, the closer the measured $D_{MSI}$ to the reference $D_{MSS}$. In other words, the distance between them is actually biased by the somatic variant fraction in the sample, in the case of a positive MSI status.

Next, a further parameter to take into account is how far the measured somatic $D_{MSI}$ may differ from the background reference $D_{MSS}$ (by a shift $p_2$ of 1bp, 2bp, 3bp, etc) at a given microsatellite locus $i$ where the patient sample may be characterized by a positive MSI status. A further variable $p_2$ may thus characterize the length distance between the main genomic alteration contributing to the measured length distribution $D_{MSI}$ (corresponding to a biological MSI event in the somatic cell formation) and the reference $D_{MSS}$ length $l_r$ at locus $i$. Applying $p_2$ to the transform function will lead part of $D_{MSS}$ (according to the somatic variant fraction ratio $p_1$) to move left or right by distance $p_2$ (direction defined by the sign of $p_2$), while the remaining part of $D_{MSS}$ (according to the germline fraction ratio 1-$p_1$) stays at the same place. In another possible embodiment, $p_2$ may be the fraction of the length difference normalized by the reference homopolymer length $l_r$ at locus $i$.

Moreover, even within the somatic cells for the same patient at a same microsatellite locus $i$, the deletion or the insertion of nucleotides in the homopolymer repeat sequences causing the MSI status are not always of the same length. For instance, in the exemplary case of an MSS reference peak length of 25bp, the MSI status may be found with repeat lengths down to 15bp, 16bp or 17bp in different cells from the same patient sample. This causes the measured length distribution $D_{MSI}$ to exhibit one peak with lower height and larger width than assumed for instance in the prior art DPF-based method from Georgiadis. A further variable $p_3$ may thus characterize the variability in the length difference between different genomic alterations contributing to the measured length distribution $D_{MSI}$. By applying $p_3$ to the transform function, the $p_1$ part of peak that moves out (by applying $p_2$) will exhibit a lower height and larger width.

This will be better understood with the examples of FIG. 3 which illustrates the contributions of different $p_1$, $p_2$ and $p_3$ values to the transformed distribution F($D_{MSS}$). In these simplified examples, germline samples follow the background reference length distribution $D_{MSS}$, $p_1$ is the ratio of somatic DNA in the sample DNA (variant fraction), $p_2$ is the length difference normalized by the reference homopolymer length at current locus (positive value means deletion and negative value means insertion) and $p_3$ means the stability of the length difference. By definition, applying $p_1$=0, $p_2$=0 and $p_3$=1 (p = {0, 0, 1}) to F will generate exactly the same distribution as original. The locus in these examples has a germline homopolymer length of 16.

As illustrated by FIG. 3a (p={1, 0, 1}): if $p_2$ equals to 0, even if $p_1$ is 100%, the transformed distribution will be the same as original. This is straightforward: if there is no mutation in somatic DNA (at least for this locus) there will be no difference no matter how variant fraction varies.

As illustrated by FIG. 3b (p={0, 1, 1}): if $p_1$ equals to 0, even if $p_2$ is 100%, the transformed distribution will be the same as original. This is also straightforward: if variant fraction is 0, the sample only has germline data so there will be no somatic difference measured no matter how much this variant will change in the somatic DNA.

As illustrated by FIG. 3c (p={1, 0.5, 1}): with a somatic variant fraction at 100% (p1=1) and a deletion of 8bp (p2=0.5 on germline reference of 16bp) the whole MSS distribution is shifted by 0.5*16=8bps to the left.

As illustrated by FIG. 3d (p={1, 0.0625, 1}): with a somatic variant fraction at 100% (p1=1) and a deletion of 1bp (p2=0.0625 on the germline reference of 16bp) the whole MSS distribution is shifted 0.0625*16=1bp to the left.

As illustrated by FIG. 3e (p={0.5, 0.5, 1}): with a somatic variant fraction at 50% (p1=0.5), half of the MSS distribution is shifted by 0.5*16=8bps (p2=0.5 on the germline reference of 16 bp) to the left, and half of the MSS distribution remains unchanged. The transformed distribution exhibits accordingly 2 peaks, each peak having half of the original germline $D_{MSS}$ peak height.

As illustrated by FIG. 3f (p={0.5, 0.0625, 1}): with a somatic variant fraction at 50%, (p1=0.5), half of the MSS distribution is shifted 0.0625*16=1bp to the left (p2=0.0625 on the germline reference of 16 bp), and half of the MSS distribution remains unchanged. The transformed distribution exhibits accordingly only 1 peak contributed from the two original peaks. However, as the 2 peaks are too close apart (only 1 bp difference) we cannot discriminate 2 separate peaks in the resulting transformed distribution.

Furthermore, in order to better fit real data without sharp peaks, a third parameter $p_3$ may be introduced to model the peak width, as there may be multiple somatic mutation events contributing to the somatic DNA such that the somatic data may exhibit a fuzzier peak than the reference background model data. As illustrated by FIG. 3g (p={0.5, 0.5, 0.5}), with a somatic variant fraction at 50% (p1=0.5), half of the MSS distribution is shifted by 0.5*16=8bps (p2=0.5 on the background reference of 16 bp) to the left, and half of the MSS distribution remains unchanged (similar to FIG. 3e)); the somatic peak attenuation parameter $p_3$ = 0.5 instead of 1 changes the shape of the somatic peak as half shorter but twice wider than the left somatic peak of FIG. 3e), while the background reference peak (right small peak in FIG. 3e)) will remain unchanged. Similarly, as illustrated by FIG. 3h (p={0.5, 0.5, 0.25}), the somatic peak attenuation parameter $p_3$ = 0.25 instead of 1 changes the shape of the somatic peak as one quarter shorter but four times wider, while the germline peak remains unchanged.

As will be apparent to those skilled in the art, the values of $p_1$, $p_2$, $p_3$ as described in the examples of FIG.3 may be interchangeable. They may also be measured as absolute values or relative to a reference. In an example (not illustrated) of using only two parameters $p_1$, $p_2$ as the variant fraction and the MSI shifted length relative to the reference MSS homopolymer length $l_r$ at locus $i$, for each possible homopolymer length $l$ ($0 \leq l \leq l_m$) F may be defined as:

$$F(D_{MSS}, p_1, p_2)(l) = (1 - p_1) * D_{MSS}(l) + p1 * D_{MSS}(l + p_2 * l_r) \; if \; 0 \leq l + p_2 * l_r \leq l_m$$

$$F(D_{MSS}, p_1, p_2)(l) = (1 - p_1) * D_{MSS}(l) \; if \; l + p_2 * l_r \leq 0 \; or \; if \; l + p_2 * l_r \geq l_m$$

where $l_r$ is the reference background model of the MSS (stable status) homopolymer length at this locus and $l_m$ is the maximum homopolymer length at this locus.

In the example of FIG.3, for each homopolymer length $l$ ($0 \leq l \leq l_m$) at one locus $i$, F may be defined as:

$$F(D_{MSS}, p_1, p_2, p_3)(l) = (1 - p_1) * D_{MSS}(l) + p_1 * p_3 * D_{MSS}\big(l_r + p_2 * l_r + p_3 * (l - l_r)\big) \; if \; 0 \leq$$
$$l_r + p_2 * l_r + p_3 * (l - l_r) \leq l_m,$$

and

$$F(D_{MSS}, p_1, p_2, p_3)(l) = (1 - p_1) * D_{MSS}(l) \; \text{else,}$$

where $l_r$ is the reference (MSS stable) homopolymer length at this locus and $l_m$ is the maximum homopolymer length at this locus.

*Inferring the model parameters to characterize MSI events*

After the parameters in the transform function model have been clearly defined, we need to measure the values of these parameters that can minimize the difference between the measured sample distribution $D_{MSI}$ and the background germline theoretical distribution $D_{MSS}$ by applying the transform function F to the background reference length distribution model $D_{MSS}$ at each locus $i$.

In a possible embodiment, at each microsatellite locus, the Genomic Data Analyzer 150 may apply a curve-fitting method by minimizing a distance metric between the transformed $D_{MSS}$ reference distribution and the measured

$D_{MSI}$ length distribution, out of possible models calculated with F according to different sets of parameters $p = \{p_1, p_2, ...\}$ to derive actual values for the parameters $p$. Various curve fitting methods may be used, for instance non-linear least-squares curve-fitting, or minimization of the sum of absolute differences (SAD). For instance, assuming that for each homopolymer locus we may have a different biological parametrization $p = \{p_1, p_2, p_3\}$, we can infer $p$ by non-linear least-squares curve-fitting:

$$\min_{p} \left\{ \sum_{l=0}^{l_m} (F(D_{MSS}, p)(l) - D_{MSI}(l))^2 \right\}$$

where $l_m$ is the maximum homopolymer length at this locus.

In another possible embodiment, for example, if there are only 2 parameters instead of 3, or if the required precision is not very high, a brute force search algorithm may alternately be applied to derive 220 the parameters instead of using a curve-fitting method.

*MSI characterization*

As mentioned above, the distribution transformation algorithm can better describe the biological principle of MSI events. In the above example, $p_1$ is defined as the ratio of somatic DNA in the patient sample, thus can be considered as how much the MSI event has spread among this patient; p2 is defined as the distance between the homopolymer length of MSI status and MSS status, thus can be considered as how severe is the MSI status (in early stage or in late stage); $p_3$ is not directly correlated with the MSI status, but, in the curve-fitting step, introducing this additional parameter $p_3$ helps to determine $p_1$ and $p_2$ more accurately out of real data.

In a possible embodiment, we may thus simply multiply $p_1$ and $p_2$ to characterize the MSI event. For each locus $i$, a local MSI score $s^i$ may then be calculated 230 as:

$$s^i = p_1^i * p_2^i$$

In a possible embodiment, for each sample, the MSI Classifier 156 may calculate 240 a raw global MSI score $S$ over all microsatellite loci, to characterize the MSI status:

$$S = \sum_{i=1}^{N} s^i = \sum_{i=1}^{N} p_1^i * p_2^i$$

where $N$ is the total number of selected loci. In another possible embodiment, $S$ may be normalized by the average MSI score of background reference (MSS) samples. Other normalization methods are also possible.

In another possible embodiment, the MSI classifier may also calculate 240 the global score $S$ by counting the number of loci with positive MSI signal, defined by a cutoff value on the local MSI status $s^i$ score for each locus $i$. Other embodiments are also possible. In general, the higher the score $S$ for a patient sample, the higher the likelihood that MSI events have happen in this sample. The MSI classifier 156 may classify MIS events according to a cut-off value, and report the MSI status as negative or positive according to how the score compares to the cut-off value.

*Exemplary Experiments*

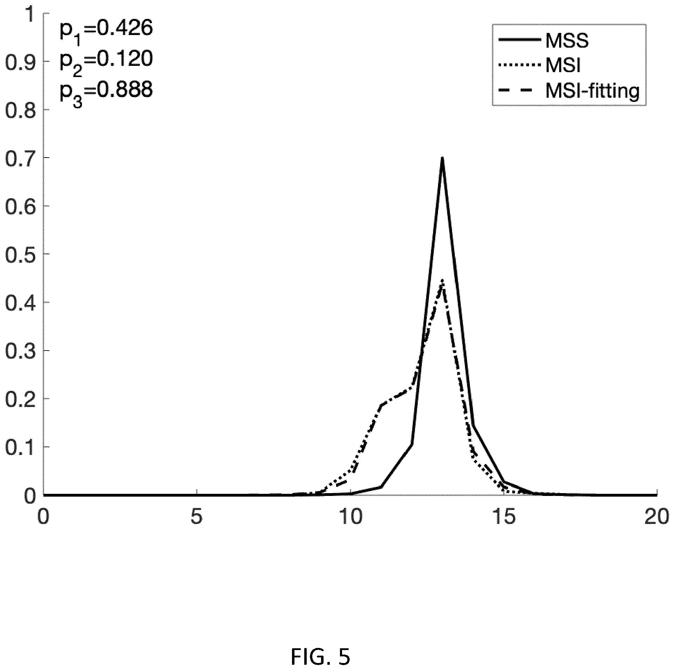

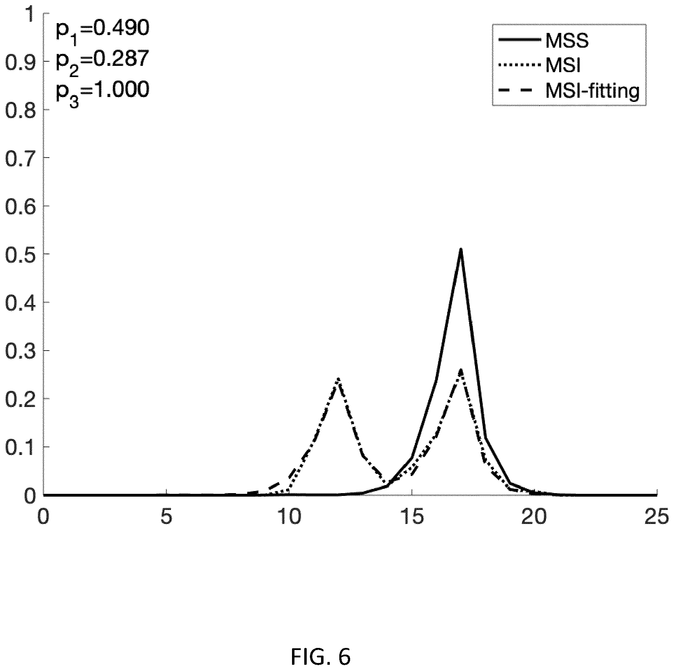

FIG. 4, FIG. 5 and FIG. 6 illustrate respectively the $D_{MSS}$ distribution, the $D_{MSI}$ distribution from the patient sample, and the MSI-fitting distribution $F(D_{MSS}, p_1, p_2, p_3)$ at 3 different microsatellite loci. In these figures, $p_1$ means the ratio of somatic DNA (variant fraction), p2 means the length difference normalized by the reference homopolymer length at current locus (positive value means deletion and negative value means insertion), and $p_3$ means the stability of the length difference. In the example of FIG. 4, the proposed method infers parameters $p_1$=0.105 for the somatic variant fraction, $p_2$=0.071 measuring a deletion of 7.1% of 14bp=0.99bp in average and $p_3$=0.965 for a more precise $p_1$ and $p_2$ fitting. The MSI score at this locus is then $p_1$*$p_2$=0.00745. Similarly, in FIG. 5, the proposed method infers parameters $p_1$=0.426 for the somatic variant fraction, p2=0.120 measuring a deletion of 12.0% of 13bp=1.6bp in average and $p_3$=0.888 for a more precise $p_1$ and $p_2$ fitting. The MSI score at this locus is then $p_1$*$p_2$=0.05112, thus significantly higher than the one of FIG. 4. Moreover, in the example of FIG.6, the proposed method infers parameters $p_1$=0.490 for the somatic variant fraction, $p_2$=0.287 measuring a deletion of 28.7% of 17bp=4.9bp in average and $p_3$=1.000 for a more precise $p_1$ and $p_2$ fitting. The MSI score at this locus is then $p_1{}^*p_2$=0.140, thus significantly higher than the ones of FIG. 4 of FIG. 5. It is therefore possible to quantify the MSI status much more accurately at each locus, no matter if the difference is huge or tiny.

Table 1 shows experimental results of the proposed MSI status analysis method benchmarked against the Promega test for a number of different tumor FFPE samples of different cancer origins (endometrial, ovarian, colon, uterus). The samples have been assayed with the capture-based kit from Sophia Genetics on a subset of microsatellite loci identified in the Salipante and the Cortes-Ciriano prior art, and sequenced with the Illumina MiSeq sequencer to provide 150bp long reads at a coverage of around 3000x at each locus. After read pre-processing and alignment, the read length distribution at each of the selected loci has been measured and fitted with LSE onto the background MSS stable length distribution model at the corresponding locus to derive three best matching parameters $p_1$, $p_2$ and $p_3$ (the same definition as described in FIG. 3, FIG. 4 and FIG.5) and a MSI score $S$ over all the selected loci has been derived for each sample according to the proposed methods. The MSI Classifier has been configured to report a positive MSI-status above a cut-off value of 0.5 on the normalized MSI score value $S$, and to report a negative MSI-status below that value. Alternately, a cut-off value of 0.07 on the raw MSI score value $S$ may be used by the Classifier. As reported in Table 1, the proposed MSI Classifier gives 100% sensitivity and 100% specificity over the tested pool of samples.

*Table 1. Comparison of the MSI status estimation with the proposed NGS-based MSI-Score Classifier Comparison and the PROMEGA reference test over a pool of different tumor samples.*

| Sample status | Sample id | MSI-Score (raw) | MSI-Score (normalized) | MSI-Score status | Promega |
|---|---|---|---|---|---|
| MSS | adenokEndocol_8792 | 0.010409 | 0 | neg | neg |
| MSS | colon_8953 | 0.026611 | 0 | neg | neg |
| MSS | colon_9215 | 0.034683 | 0.037613 | neg | neg |
| MSS | colon_9226 | 0.024668 | 0 | neg | neg |
| MSS | colon_9240 | 0.0033 | 0 | neg | neg |
| MSS | colon_9247 | 0.025759 | 0 | neg | neg |
| MSS | colon_9315 | 0.04469 | 0.157924 | neg | neg |
| MSS | colon_9316 | 0.0506 | 0.22897 | neg | neg |
| MSS | colon_9318 | 0.025992 | 0 | neg | neg |
| MSS | colon_9321 | 0.042781 | 0.134967 | neg | neg |
| MSS | colon_9328 | 0.053061 | 0.258568 | neg | neg |
| MSS | colon_9331 | 0.0278 | 0 | neg | neg |
| MSS | colon_9339 | 0.004787 | 0 | neg | neg |
| MSS | colon_9340 | 0.024609 | 0 | neg | neg |
| MSS | colon_9417 | 0.043817 | 0.14743 | neg | neg |
| MSS | colon_9467 | 0.048415 | 0.202702 | neg | neg |
| MSS | endometre_8510 | 0.063039 | 0.378521 | neg | neg |
| MSS | endometre_8729 | 0.016525 | 0 | neg | neg |
| MSS | endometre_9084 | 0.022159 | 0 | neg | neg |
| MSS | ovaire_8969 | 0.018919 | 0 | neg | neg |
| MSS | ovaire_9025 | 0.050016 | 0.221957 | neg | neg |
| MSI | HD_701 | 3.531905 | 1 | pos | pos |

(continued)

| Sample status | Sample id | MSI-Score (raw) | MSI-Score (normalized) | MSI-Score status | Promega |
|---|---|---|---|---|---|
| MSI | adenokEndometre_9304 | 0.357279 | 1 | pos | pos |
| MSI | endometre_7802 | 0.468486 | 1 | pos | pos |
| MSI | endometre_8283 | 0.298747 | 1 | pos | pos |
| MSI | endometre_8396 | 0.33278 | 1 | pos | pos |
| MSI | endometre_9179 | 0.513835 | 1 | pos | pos |
| MSI | endometre_9396 | 1.309561 | 1 | pos | pos |
| MSI | endometre_9462 | 0.781316 | 1 | pos | pos |
| MSI | endometre_9497 | 0.534864 | 1 | pos | pos |
| MSI | endometre_9500 | 0.854427 | 1 | pos | pos |
| MSI | uterus_9038 | 0.079785 | 0.579853 | pos | pos |

As reported in Table 2, the proposed NGS-based MSI Classifier also exhibits a lower limit of detection (LOD) compared to the Promega tests. For this experiment, 8 different dilutions of MSI-confirmed DNA sample and MSS-confirmed DNA samples have been tested, respectively comprising 1% (0.5ng) to 90% (45ng) of MSI-confirmed DNA in a sample of 50ng mixed DNA content. On sample mixtures, the proposed NGS-based MSI Classifier provides the MSI status with down to 1% MSI tumor content mixed with 99% MSS content, while the Promega test only provides satisfactory results above a LOD of 20%.

*Table 2 Comparison of limits of detection for the PROMEGA reference test and the proposed NGS-based MSI-Score Classifier.*

| Sample status | Sample id | MSI-Score (raw) | MSI-Score (normalized) | MSI-Score status | Promega |
|---|---|---|---|---|---|
| 1%MSI+99%MSS | LOD_01 | 0.1095 | 0.8815435 | pos | neg |
| 2%MSI+98%MSS | LOD_02 | 0.1138085 | 0.925396 | pos | neg |
| 5%MSI+95%MSS | LOD_05 | 0.144436 | 1 | pos | doubtful |
| 10%MSI+90%MSS | LOD_10 | 0.187231 | 1 | pos | doubtful |
| 20%MSI+80%MSS | LOD_20 | 0.345927 | 1 | pos | pos |
| 30%MSI+70%MSS | LOD_30 | 0.4822865 | 1 | pos | pos |
| 50%MSI+50%MSS | LOD_50 | 0.649463 | 1 | pos | pos |
| 90%MSI+10%MSS | LOD_90 | 1.2704465 | 1 | pos | pos |

**Claims**

1.   A method for determining a microsatellite instability (MSI) status of a patient comprising:

- obtaining DNA fragments from a biological DNA sample from a patient, the sample comprising cells from a solid tissue or a bodily fluid;
- sequencing the DNA fragments with a high throughput sequencing technology to obtain a plurality of data reads for each DNA fragment;
- aligning the data reads to a reference genome DNA sequence comprising a predefined set of N microsatellite genomic loci;

the method further comprising:

- at each microsatellite genomic locus i in the predefined set of microsatellite genomic loci, measuring a patient sample distribution $D_{MSI}$ of the nucleotide repeat lengths for the set of aligned data reads mapped to the reference genome DNA sequence at the microsatellite locus $i$ and estimating a local MSI status score $s_i$ as a function of the difference of the measured patient sample distribution $D_{MSI}$ of the nucleotide repeat lengths relative to a reference background distribution model $D_{MSS}$ of the nucleotide repeat lengths at the microsatellite genomic locus $i$;
- determining a global MSI score $S$ to characterize the MSI status of the patient sample as a function of all estimated local MSI status scores $s_i$, $i$=1 to N,

**characterized in that**

- each microsatellite genomic locus in the predefined set is a mono-homopolymer repeat of a single nucleotide;
- each microsatellite genomic locus in the predefined set has a reference repeat length of at least 13 nucleotides (13bp) and at most 25 nucleotides (25bp);

2. The method of claim 1, further **characterized in that** the local MSI status score $s_i$ is calculated as a function of at least two independent scalar parameters $p_1$, $p_2$ of a multi-parametric function $F(D_{MSS}, p_1, p_2)$ of the reference background distribution model $D_{MSS}$ of the nucleotide repeat length such that $D_{MSI} = F(D_{MSS}, p_1, p_2)$ at the microsatellite genomic locus $i$.

3. The method of claim 2, further **characterized in that** the local MSI status score $s_i$ is calculated as a function of at least three independent scalar parameters $p_1$, $p_2$, $p_3$ of a multi-parametric function $F(D_{MSS}, p_1, p_2, p_3)$ of the reference background distribution model $D_{MSS}$ of the nucleotide repeat length such that $D_{MSI} = F(D_{MSS}, p_1, p_2, p_3)$ at the microsatellite genomic locus $i$.

4. The method of claims 2 or 3, in which the DNA sample comprises a mixture of somatic cells DNA and normal cells DNA in an undetermined variant fraction which is variable from one patient sample to another patient sample, further **characterized in that** at least one of the independent scalar parameters is the variant fraction $p_1$ measuring the ratio of somatic DNA content relative to the total DNA content in the patient sample.

5. The method of claims 2 or 3, further **characterized in that** at least one of the independent scalar parameters is a microsatellite length shift value p2 **characterizing by** how many insertions or deletions the somatic microsatellite length has shifted in the somatic DNA normalized relative to the microsatellite stable status length at the microsatellite genomic locus $i$.

6. The method of claim 5, further **characterized in that** the multi-parametric function $F(D_{MSS}, p_1, p_2)$ is defined for each possible length value $l$ at the microsatellite locus $i$, $0 \leq l \leq lm$ as:

$$F(D_{MSS}, p_1, p_2)(l) = (1 - p_1) * D_{MSS}(l) + p1 * D_{MSS}(l + p_2 * l_r)\ if\ 0 \leq l + p_2 * l_r \leq l_m$$

$$F(D_{MSS}, p_1, p_2)(l) = (1 - p_1) * D_{MSS}(l)\ if\ l + p_2 * l_r \leq 0\ or\ if\ l + p_2 * l_r \geq l_m$$

where $l_r$ is the reference stable homopolymer length at this locus and $l_m$ is the maximum homopolymer length at this locus.

7. The method of claim 5, further **characterized in that** at least one of the independent scalar parameters is a microsatellite length stability value p3 characterizing how variable the somatic microsatellite length shift is in the somatic DNA content.

8. The method of claim 7, further **characterized in that** the multi-parametric function $F(D_{MSS}, p_1, p_2, p_3)$ is defined for each possible length value $l$ at the microsatellite locus $i$, $0 \leq l \leq lm$ as

$$F(D_{MSS}, p_1, p_2, p_3)(l)$$

$$= (1 - p_1) * D_{MSS}(l) + p_1 * p_3 * D_{MSS}(l_r + p_2 * l_r + p_3 * (l - l_r)) \; if \; 0 \leq l_r$$

$$+ p_2 * l_r + p_3 * (l - l_r) \leq l_m$$

$$F(D_{MSS}, p_1, p_2, p_3)(l) = (1 - p_1) * D_{MSS}(l) \; else,$$

where $l_r$ is the reference stable homopolymer length at this locus and $l_m$ is the maximum homopolymer length at this locus.

9. The method of claims 2 to 8, further **characterized in that** the independent scalar parameters p={$p_1$, $p_2$} or p={$p_1$, $p_2$, $p_3$} at locus $i$ are inferred by minimizing the difference between the measured patient distribution $D_{MSI}$ and the predicted patient distribution $F(D_{MSS}, p)$ at locus $i$.

10. The method of claim 9, further **characterized in that** minimizing the difference between the measured patient distribution $D_{MSI}$ and the predicted patient distribution $F(D_{MSS}, p)$ comprises minimizing the sum of the absolute differences (SAD) or the least square error (LSE) between the measured and the predicted distributions.

11. The method of claim 10, further **characterized in that** minimizing the difference between the measured patient distribution $D_{MSI}$ and the predicted patient distribution $F(D_{MSS}, p)$ $p_2$,) comprises assuming a constant value for at least one of the parameters p=$p_1$,p2,... and brute force searching for all possible values for the other variable parameters such that such that $D_{MSI} = F(D_{MSS}, p)$.

12. The method of claims 2 to 11, further **characterized by** estimating the local MSI status score as

$$s^i = p_1^i * p_2^i.$$

13. The method of claim 12, further **characterized by** determining the global MSI score as a raw MSI score $S$ calculated as the sum of the local MSI status scores over the N loci, or a normalized MSI score calculated as the sum of the local MSI status scores normalized to the highest local MSI score over the N loci, and/or an MSI score count calculated as the number of loci in the set of N loci where the local MSI score is over a predefined threshold.

14. The method of claim 13, further **characterized in** determining the microsatellite instability (MSI) status of the patient as positive if the global MSI score $S$ over the N loci is above a predefined cutoff value, and negative if the global MSI score $S$ over the N loci is below the predefined cutoff value.

50

DNA samples     Ligation adapters

**Fragmentation**

DNA fragments

**Ligation** — 100

Library of DNA-
adapter products

**Amplification** — 110

PCR duplicates

**Sequencing** — 120

*Laboratory*

Raw NGS
sequencing reads
(FASTQ)

151

**Pre-processing** — 151

Pre-processed reads (FASTQ)

**Sequence alignment** — 152

Alignment data (BAM)

**Variant calling** — 153     **MS Loci Analyzer** — 155

Variants (VCF)

**Variant annotation** — 154     **MSI Classifier** — 156 150

*Genomic Data Analyzer*

Genomic variant report     **FIG.1**     MSI status report

Alignment data (BAM)
At locus 1, 2, … , l, … , N

$D_{MSS}$ length distribution models at locus 1, 2, … , l, … , N — 205

**210** — Measure $D_{MSI}(l)$ length distribution in tumor sample

**220** — Infer parameters p1, p2, … of tumor sequence mutation function such that $D_{MSI}(l) = F(D_{MSS}(l),p1,p2,...)$

**230** — Calculate local MSI score $S_l$ at locus $l$

MSI-score At locus 1, 2, … , l, … , N

**240** — Calculate global MSI-score S for tumor sample

MSI status report

FIG. 2

FIG. 3

FIG. 4

FIG. 5

FIG. 6

Europäisches
Patentamt

European
Patent Office

Office européen
des brevets

## EUROPEAN SEARCH REPORT

Application Number

EP 20 15 6032

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/002621 A2 (HOFFMANN LA ROCHE [CH]; ROCHE DIAGNOSTICS GMBH [DE] ET AL.) 2 January 2020 (2020-01-02) * abstract * * paragraph [0066] - paragraph [0085]; table 2 * * paragraph [0130] - paragraph [0143] * | 1-14 | INV. G16B20/20 G16B40/30 |
| X | US 2020/032332 A1 (ZHANG ZHIHONG [CN] ET AL) 30 January 2020 (2020-01-30) * abstract * * paragraph [0009] - paragraph [0010]; table 1 * * paragraph [0019] - paragraph [0033] * * paragraph [0047] - paragraph [0059] * | 1-14 | |
| A | LAURA G. BAUDRIN ET AL: "Molecular and Computational Methods for the Detection of Microsatellite Instability in Cancer", FRONTIERS IN ONCOLOGY, vol. 8, no. 621, 12 December 2018 (2018-12-12), XP055678952, DOI: 10.3389/fonc.2018.00621 * abstract * * page 5 - page 8 * | 1-14 | TECHNICAL FIELDS SEARCHED (IPC) G16B |

The present search report has been drawn up for all claims

| Place of search | Date of completion of the search | Examiner |
|---|---|---|
| Munich | 13 July 2020 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 15 6032

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2020

| Patent document cited in search report | Publication date | Patent family member(s) | Publication date |
|---|---|---|---|
| WO 2020002621 A2 | 02-01-2020 | NONE | |
| US 2020032332 A1 | 30-01-2020 | CN 106755501 A | 31-05-2017 |
| | | EP 3597769 A1 | 22-01-2020 |
| | | JP 2020505925 A | 27-02-2020 |
| | | US 2020032332 A1 | 30-01-2020 |
| | | WO 2018137678 A1 | 02-08-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20140052381 A, Utiramerur
- EP 2019065777 W, Lin and Zu
- WO 2013153130 A, Lambrechts
- WO 2017112738 A, Perry
- WO 2018037231 A, Burn
- WO 2019108807 A, Georgiadis
- WO 2017220508 PCT

### Non-patent literature cited in the description

- **BAUDRIN et al.** Molecular and computational methods for the detection of microsatellite instability in cancer. *Frontiers in Oncology,* December 2018, https://doi.org/10.3389/fonc.2018.00621
- Microsatellite instability detection by next generation sequencing. *Clinical Chemistry,* 2014, vol. 60 (9), 1192-1199
- **HAUSE et al.** Classification and characterization of microsatellite instability across 18 cancer types. *Nature Medicine,* 2016, vol. 22, 1342-1350
- **CORTES-CIRIANO et al.** A molecular portrait of microsatellite instability across multiple cancers. *Nature Communications*
- **SINGHI.** Real-time targeted genome profile analysis of pancreatic ductal adenocarcinomas identifies genetic alterations that might be targeted with existing drugs or used as biomarkers. *gastrology,* 2019, vol. 156, 2242-2253
- **NIU et al.** MSIsensor: microsatellite instability detection using paired tumor-normal sequence data. *Bioinformatics,* 2014, vol. 30 (7), 1015-1066